(19) Europäisches Patentamt
European Patent Office
Office européen des brevets

(11) Publication number : 0 542 565 A1

(12) EUROPEAN PATENT APPLICATION

(21) Application number : 92310384.0

(22) Date of filing : 13.11.92

(51) Int. Cl.[5] : A61K 31/70, A61K 31/505, A61K 31/165, A61K 31/135, // (A61K31/70, 31:165, 31:135), (A61K31/505, 31:165, 31:135)

(30) Priority : 13.11.91 US 791765

(43) Date of publication of application : 19.05.93 Bulletin 93/20

(84) Designated Contracting States : AT BE CH DE DK ES FR GB GR IE IT LI LU MC NL PT SE

(71) Applicant : GENSIA PHARMACEUTICALS, INC.
11025 Roselle Street
San Diego, CA 92121 (US)

(72) Inventor : Tuttle, Ronald
2704 Vista Del Sombrado
Escondido, California 92025 (US)

(74) Representative : Sexton, Jane Helen et al
J.A. Kemp & Co. 14 South Square Gray's Inn
London WC1R 5LX (GB)

(54) **Pharmacological stress testing for coronary artery disease.**

(57) A method of pharmacological stress testing for diagnosis of coronary artery disease comprising administration of adenosine or an adenosinergic agent in conjunction with a synthetic catecholamine offers advantages over conventional exercise stress tests.

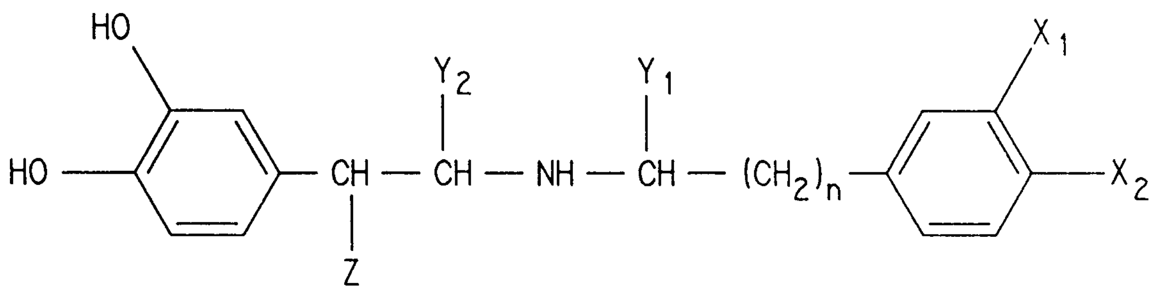

EP 0 542 565 A1

Publications and other reference materials referred to herein are incorporated herein by reference and are numerically referenced in the following text and respectively grouped in the appended Bibliography which immediately precedes the claims.

This invention is directed to improving the sensitivity, safety, and patient acceptability of pharmacological stress testing.

Coronary artery disease (CAD) is responsible for more death and disability than any other disease. Thus, an invention that contributes to an improved method of detection and evaluation of CAD will have a positive impact on public health. An important test method for detecting CAD is exercise tolerance testing (ETT). However, many patients with suspected CAD are incapable of an ETT. Moreover, even in patients who can exercise, exercise may interfere with the optimum application of one of the most sensitive, safe and convenient instruments for the detection of CAD, echocardiography.

Pharmacological stress testing with adenosine, or an adenosine transport inhibitor (ATI) or a catecholamine may provide an alternative to exercise. Pharmacological stress testing with either adenosine, an ATI or catecholamine has been reported (1, 2, 3, 4, 5). The utility of those reported methods of pharmacological stress testing in the detection and evaluation of CAD has been limited by their sensitivity and safety, and also by the discomforting side effects reported for those methods.

A method for improving the sensitivity and safety of pharmacological stress tests is disclosed herein.

An improved method of diagnosing and evaluating coronary artery disease comprises the administration of either adenosine or an adenosinergic agent, such as adenosine transport inhibitor (ATI), in conjunction with an appropriate catecholamine. This method advantageously offers improved sensitivity and safety in the diagnosis of CAD, and reduced side effects compared to reported methods of pharmacological stress testing.

According to one aspect, when CAD is present, pretreatment with a catecholamine is used to predispose the heart to myocardial ischemia which is then induced by administration of adenosine or an adenosinergic agent. According to a second aspect, when CAD is present, the heart is predisposed to catecholamine-induced ischemia by pretreating the patient with adenosine or an adenosinergic agent, such as an ATI. According to either aspect, the catecholamine administered is a synthetic catecholamine (i.e., not one of the natural catecholamines produced by and released in the body such as dopamine, norepinephrine, or epinephrine). Suitable synthetic catecholamines are those which elicit cardiovascular effects which include increases in heart rate, in cardiac contractility, in cardiac output and in systolic blood pressure. Preferably, the catecholamine elicits cardiovascular effects which are similar to those cardiovascular effects elicited by aerobic exercise. More preferably, the catecholamine administered is a synthetic catecholamine having a chemical structure such as that depicted by Figure 1.

According to a first aspect, myocardial requirements for blood flow are elevated in a progressive and stepwise manner by the controlled intravenous infusion of synthetic catecholamine. Alternatively, the catecholamine may be administered iontophoretically. During each of these elevated steps, a fixed dose of adenosine or an adenosinergic agent is intravenously administered over a fixed interval of time. If the dose of adenosine or adenosinergic agent causes myocardial ischemia, the diagnosis of CAD is made. Suitable means of detection include electrocardiography or echocardiography. If the dose of adenosine or adenosinergic agent does not cause myocardial ischemia during maximal catecholamine-induced elevations in myocardial blood flow requirements, CAD is ruled out. The magnitude of the catecholamine-induced elevation in myocardial blood flow requirement may be conveniently gauged by the continuous recording of the patient's heart rate. In addition to providing a diagnostic yes/no answer on the presence of CAD, the method according to this aspect allows the evaluator to make an assessment of disease severity and, thus, as appropriate, patient prognosis. For example, if the dose of adenosine or adenosinergic agent provokes ischemia at step one, (e.g. when the catecholamine has elevated the heart rate to 90 beats/minute), the coronary artery disease would be evaluated as more severe than if the dose of adenosine or adenosinergic agent did not provoke ischemia until step three (e.g. when the catecholamine has elevated the heart rate to 150 beats/minute).

According to a second aspect, the patient is given either intravenously or orally an adenosinergic agent, preferably an ATI, from 5 to 60 minutes before commencing administration of catecholamine to induce the catecholamine-induced elevations in myocardial blood flow requirements. According to this aspect, adenosine *per se* is not administered. The diagnostic/prognostic information obtained using this second aspect are similar to that obtained using the fixed adenosine dose.

## Definitions

As used herein, the following terms have the following meanings unless expressly stated to the contrary.

The term "adenosinergic agent" refers to an agent which increases extracellular levels of adenosine. One class of adenosinergic agents comprise adenosine transport inhibitors, that is agents which increase local extracellular adenosine levels by preventing adenosine transport.

The term "sensitivity" refers to the ability of a test (or diagnostic method) to recognize the presence of a particular disease in a patient or a population of patients.

The term "specificity" refers to the ability of a test (or diagnostic technique) to distinguish those patients who do not have the particular disease from those who do have the disease.

## BRIEF DESCRIPTION OF THE DRAWINGS

Figure 1 depicts a general formula for preferred synthetic catecholamines for use according to the present invention.

## DETAILED DESCRIPTION OF THE INVENTION

### I. CARDIAC EFFECTS OF ADENOSINE AND ADENOSINERGIC AGENTS

Dipyridamole, which is a known ATI, and adenosine have been reported to cause changes in the blood flow pattern (1, 6, 7). Dipyridamole is reported to exert its effect on the blood flow pattern by blocking transport of adenosine across cell membranes (6). Blocking transport increases the concentration of endogenous adenosine released from myocardial cells.

Non-cardiac side effects (e.g. headache, dizziness, nausea, flushing, and paresthesia) have been reported to be more common with adenosine than with dipyridamole, because when adenosine is intravenously infused it is distributed throughout the circulation. Although dipyridamole (or ATIs) are also be distributed throughout the circulation, the effect of such agents would be primarily on the coronary vasculature, since the heart is the organ with highest rate of adenosine release. Even though the above-noted side effects have been reported to be more common with adenosine than with dipyridamole, for certain indications, adenosine may still be preferred over dipyridamole, due to its fast onset of action, and extremely short duration of action. Wilson et al (8) reported that adenosine produced a maximal effect on the coronary vasculature within two minutes after administration was begun, and that the effect of adenosine lasted for only about two minutes after its administration was stopped. The maximal effect of dipyridamole was reported to occur within four minutes of when administration began, but the effects of dipyridamole were reported to persist for twenty to forty minutes after its administration was stopped.

### II. GENERAL ASPECTS CONCERNING METHODS USED IN THE DETECTION OF CAD

Four frequently used methods of detection and evaluation of coronary artery disease are:

- Coronary Arteriography
- Stress - Radionuclide Imaging
- Stress - Echocardiographic Imaging
- Stress - Electrocardiography (ECG)

Coronary arteriography provides anatomical evidence of CAD, and it is considered the "gold standard" by which all other methods are judged. The other three methods are indirect functional tests, and they require the heart to be stressed either by exercise or pharmacologically. While arteriography provides the most definitive evidence of CAD, it is invasive and expensive. Consequently one of the other methods usually is employed to screen out patients who are unlikely to have the disease before coronary arteriography is used.

The ECG and Echocardiographic imaging methods rely on the induction of myocardial ischemia to make a diagnosis of CAD. Radionuclide imaging relies on inducing changes in the myocardial pattern of blood flow. If extreme changes in the blood flow pattern are induced, myocardial ischemia will occur. However with radionuclide imaging it is unnecessary to go to this extreme.

### III. CAD DIAGNOSIS WITH ADENOSINE OR ATI WHEN USED WITH RADIONUCLIDE IMAGING

Flow of blood through a tissue is equal to the perfusion pressure divided by the vascular resistance to flow:

$$\text{Flow} = \frac{\text{Pressure}}{\text{Resistance}}$$

The coronary vasculature has a great capacity to auto-regulate myocardial blood flow. For example, flow to myocardium supplied by normal coronary arteries can increase by as much as 500-600% during exercise. This increase in blood flow is accomplished at least in part by a decrease in coronary vascular resistance. The

degree to which flow can be increased by decreasing resistance is known as ("coronary reserve"). Coronary reserve will be reduced in regions of the myocardium which are supplied by disease-obstructed arteries, because much of the reserve has been used to compensate for the reduced perfusion pressured[1]. However, in myocardium supplied by disease-free arteries the reserve capacity is still available. Consequently, an agent having a strong vasodilating effect can greatly reduce vascular resistance and, thus, will increase flow to regions supplied by normal arteries. Since arterioles in regions supplied by the diseased arteries may be almost fully dilated before the vasodilating agent is given, they will have little or no remaining flow reserve.

Adenosine has been reported to be a profound coronary artery vasodilator. When extracellular myocardial concentrations of adenosine are elevated in a patient without CAD, such as by giving the patient adenosine or an adenosinergic agent (for example an ATI), a large and homogeneous increase in coronary blood flow occurs. On the other hand, when CAD is present, little coronary reserve remains in the regions of myocardium supplied by the disease-obstructed vessels. Consequently, administration of adenosine or an adenosinergic agent can cause little further decrease in resistance and, therefore, its administration will result in little or no increase in blood flow. In regions of myocardium supplied by relatively disease-free vessels, administration of adenosine or an adenosinergic agent will cause relatively large decreases in vascular resistance and the resulting increases in flow. In the presence of CAD, an elevation of adenosine levels will produce a heterogeneous rather than homogeneous change in myocardial perfusion, depending on the state of the arteries supplying a particular region (i.e. whether diseased or not). This heterogeneous change in perfusion pattern is detectable using radionuclide imaging.

The most frequently used radionuclide imaging agent is thallium-201. Thallium-201 uptake by a region of the myocardium is proportional to myocardial blood flow in that region. Thallium-201 is a gamma emitter; an image of myocardial perfusion is obtained by positioning a gamma camera over the patient's chest. The image obtained using Thallium-201 imaging will reveal flow heterogeneities in response to adenosine administration if significant CAD is present. Adenosine-induced flow heterogeneity has been reported to be a sensitive method for the detection and evaluation of CAD (9).

Although radionuclide imaging may not be as hazardous a procedure to a patient as is coronary arteriography, it can only be performed in hospitals having nuclear medicine facilities. It is also an expensive and time-consuming procedure. For these reasons, either electrocardiography or echocardiography are often performed before or in preference of radionuclide imaging.

IV. <u>DETECTION OF CAD WITH ADENOSINE OR ADENOSINERGIC AGENTS USED WITH ELECTROCARDIOGRAPHY AND ECHOCARDIOGRAPHY</u>

A first line test for CAD is exercise electrocardiography. This test is generally known as an exercise tolerance test (ETT). An ETT is performed by having a patient exercise on a treadmill or stationary bicycle while his electrocardiagram (ECG) is recorded simultaneously. If significant CAD is present, the stress of exercise will cause those portions of the myocardium supplied by the disease vessels to become ischemic. The resulting myocardial ischemia is detected by a characteristic change in the ECG pattern called ST segment depression.

The ECG is a relatively insensitive means of detecting myocardial ischemia. In order to produce an unambiguous sign of ischemia on the ECG, a considerable myocardial mass must become ischemic. A dose of adenosine or of an ATI large enough to consistently produce signs of ischemia detectably by ECG in patients with CAD is higher than that which can safely be tolerated by such patients. Thus, neither adenosine nor ATIs would be useful for detecting and evaluating CAD using ECG. An important objective of this invention is to improve the sensitivity and safety of adenosine and ATIs in causing detectable myocardial ischemia to the point where ECGs will be useful in detecting CAD.

Levels of myocardial ischemia that would not be detected by ECG can be detected by echocardiography. Thus, it has been possible with echocardiography, to use doses of adenosine or the ATI, dipyridamole, small enough to be tolerated to diagnose CAD. However, even using echocardiography, the usefulness of adenosine or ATIs alone to detect CAD is limited. For example, Nguyen <u>et</u> <u>al</u> (10) identified 16 of 20 CAD patients using adenosine administration in combination with thallium-201 imaging. However, when those researchers used echocardiography in combination with adenosine doses only 2 of the 20 CAD patients were identified as having CAD. Therefore, it is also an important objective of the invention to improve the sensitivity of adenosine and ATIs when used with echocardiography testing for CAD.

[1]If significant disease is present in a vessel, the vessel will be obstructed to the point that perfusion pressure distal to the obstruction is greatly reduced. To maintain myocardial blood flow to a region supplied by an obstructed vessel, coronary vascular resistance is reduced.

## V. POTENTIATING ADENOSINE-INDUCED MYOCARDIAL ISCHEMIA

### A. Supply side Ischemia

To enable the use of either electrocardiography or echocardiography for the detection of CAD, detectable myocardial ischemia must be elicited when significant disease is present. Myocardial ischemia occurs when the myocardium's demand for blood flow exceeds the blood flow availability. Thus, ischemia can be elicited by changes either in blood flow demand or blood flow supply. As blood supply decreases ischemia increases, and as blood demand increases ischemia increases:

$$\text{ISCHEMIA} = \frac{\text{DEMAND}}{\text{SUPPLY}}$$

Adenosine can induce ischemia by decreasing blood flow supply. An adenosine-induced decrease in blood flow to myocardium supplied by disease-obstructed arteries results from a phenomenon known as "coronary steal". coronary steal is an extreme manifestation of adenosine's coronary vasodilating properties. As previously noted, portions of myocardium supplied by diseased vessels will reduce coronary reserve due to the reduced perfusion pressure. The magnitude of vasodilation that can be induced by adenosine is proportional to the remaining coronary reserve. Since coronary reserve is larger in regions of myocardium supplied by relatively normal vessels, than it is in regions of myocardium supplied by diseased vessels, administration of adenonine will cause a (proportionally) larger drop in vascular resistance of areas supplied by normal vessels. Consequently, blood flow will be diverted to regions supplied by normal (non-diseased) vessels at the expense of flow to regions supplied by disease-obstructed vessels. Thus, the myocardial ischemia caused by adenosine administration is ischemia caused by reduced blood flow supply. It will be referred to herein subsequently as "supply side ischemia".

### B. Demand Side Ischemia

In contradistinction to the supply side ischemia caused by adenosine administration, the ischemia caused by catecholamine administration results from an increase in demand of the myocardium for blood flow. Ischemia caused by an increase in demand will subsequently be referred to herein as "demand side ischemia".

Certain synthetic catecholamines have been reported to increase the demand for myocardial blood flow by elevating heart rate, cardiac contractility, and systolic blood pressure.

These catecholamines include those having the structure depicted in Figure 1.

By elevating cardiac rate, contractility and systolic pressure in a patient with CAD, just prior to the administration of standardized doses of adenosine, the stage will be set for adenosine to induce ischemia. Ischemia will result from both the demand and supply side. Consequently, the dose of adenosine needed to produce ischemia will be reduced. As the dose of adenosine is reduced, a reduction in the non-cardiac side effects of adenosine will accompany the reduction in adenosine dose.

### C. Demand and Supply Side Ischemia Caused by Catecholamine Administration Followed by Adenosine (or Adenosinergic Agent) Administration

By inducing demand and supply side ischemia (i.e. working both sides of the ischemia equation), the portion (or proportion) of myocardium that can be safely made ischemic with the injection of adenosine or an adenosinergic agent is expanded. Expansion of the portion of myocardium made ischemic by the administration of adenosine or adenosinergic agent will enhance the ability to detect CAD (i.e. sensitivity) using either electrocardiography and echocardiography. Suitable adenosinergic agents for use according to this method of the present invention have a short half life or duration of action. Preferred adenosinergic agents have a half life of less than 5 minutes. Especially preferred are adenosinergic agents having a half life of less than 1 minute.

According to the methods of the present invention, the catecholamine-induced increase in myocardial blood flow demand is followed by continuous monitoring of the patient's heart rate. The heart rate may be conveniently monitored using the ECG. According to one embodiment of the present invention, the test to determine presence of CAD is done in steps, using incremental increases in catecholamine-induced blood flow demand in conjunction with administration of a fixed dose of adenosine or an adenosinergic agent. For example, step 1 could comprise an intravenous infusion of the synthetic catecholamine at a rate required to raise the heart rate (over the resting rate) by 20 beats/minute. Steps 2, 3 and 4 could then comprise additional intravenous infusions of the catecholamine that would raise the rate by 40, 60 and 80 beats/minute, respectively, over the initial rate coupled with additional infusions of the fixed dose of adenosine or adenosinergic agent. With each stepwise increase in heart rate, a standardized dose of adenosine or adenosinergic agent (which would

range from X to Y mg/kg) would be intravenously administered over a predetermined time (seconds). During the administration of adenosine or adenosinergic agent, the catecholamine infusion would continue uninterrupted. If the administration of adenosine or adenosinergic agent precipitated myocardial ischemia, the catecholamine infusion would be immediately stopped. The resulting ischemia would be positive evidence that the patient had CAD. If adenosine or the adenosinergic agent failed to precipitate ischemia at any of the levels of catecholamine infusion, that result would indicate that significant CAD was absent.

Optionally, the catecholamine may be administered using a closed loop drug delivery system, that is, a drug delivery system in which drug is delivered or delivery of drug is stopped in automatic response to feedback of a physical signal (or response), such as heart rate for example in the present instance. These closed loop systems may include closed loop iontophoretic drug delivery systems as well as closed loop intravenous infusion drug delivery systems. Such closed loop systems may advantageously administer precise amounts of drug to the patient so that a desired response level may be maintained or, optionally, increased or decreased. The administration of drug by the closed loop device may be pulsitile or constant rate. By using pulsitile delivery, it may be possible to deliver less total drug and yet get the same response than with constant delivery of drug.

The use of the synthetic catecholamine in combination with adenosine or an adenosinergic agent will result in reduction of the dose of adenosine or adenosinergic agent required to cause myocardial ischemia because myocardial demand already will be elevated due to administration of catecholamine at the point when adenosine or the adenosinergic agent is administered (which results in reduced supply). In addition, the combined administration of catecholamine and adenosine or the adenosinergic advantageously affects the distribution of the intravenously administered adenosine or adenosinergic agent. Administration of the catecholamine will cause a greater proportion of the dose of adenosine or adenosinergic agent to be distributed to the heart. Moreover, a greater proportion of this now enhanced cardiac fraction of adenosine or adenosinergic agent will be distributed to portions of the myocardium supplied by non-diseased coronary arteries, and, thus, will potentiate coronary steal. The changed distribution of adenosine or the adenosinergic agent will further reduce dose of adenosine or adenosinergic agent needed for diagnosis of CAD, and, thus, will substantially reduce the non-cardiac side effects of adenosine or the adenosinergic agent. Some of the non-cardiac side effects that will be reduced include: nausea, dyspnea, and flushing.

These diagnostically advantageous changes in the distribution of the intravenously administered adenosine or adenosinergic agent result from the effects of the catecholamine on coronary fraction and on the intramyocardial distribution of blood flow. Since the synthetic catecholamine increases myocardial demand by elevating heart rate, cardiac contractility, and systolic blood pressure, coronary blood flow will rise in proportion to the increase in myocardial demand. Therefore, the fraction of the cardiac output being delivered to the heart as opposed to the peripheral circulation will increase, and Consequently a greater fraction of the dose of adenosine or adenosinergic agent will be carried to the heart than would occur if the synthetic catecholamine were not simultaneously administered. If CAD is present, the regions of myocardium supplied by the diseased (obstructed) arteries will experience a smaller catecholamine-induced increase in blood flow than will the regions of myocardium supplied by relatively unobstructed arteries. Consequently, more adenosine or adenosinergic agent will be carried to the latter regions that have less CAD, thereby, initiating coronary steal sooner than if adenosine or the adenosinergic agent were given alone, that is, without the benefit of the catecholamine-induced changes on distribution of the intra-myocardial blood flow.

An important aspect of this invention is that the combined administration of catecholamine and adenosine or an adenosinergic agent (such as an ATI) will cause a large enough mass of the myocardium to become ischemic to be detectable with ECG. Large areas of myocardium cannot be made ischemic by administration of adenosine or adenosinergic agent alone without risking cardiovascular collapse, because the contractlle function of the myocardium is impaired when it becomes ischemic. If too great a proportion of the myocardium becomes ischemic, stroke volume (the volume of blood pumped per beat) will fall to the point that cardiac output is too low to maintain blood pressure and, thus, collapse and death may result. However, administration of the synthetic catecholamine with adenosine or adenosinergic agent will increase the contractile function of, regions of the myocardium supplied by relatively unobstructed arteries. The positive effect of the synthetic catecholamine on contractile function in the non-ischemic regions of the myocardium will compensate for the ischemia-induced depression of contractility in regions supplied by diseased arteries, and, thus, will prevent stroke volume from falling to dangerously low levels.

Adenosine doses that are just adequate to detect CAD by radionuclide imaging have been reported to produce from a 10 to 13% incidence of heart block (10). Such partial heart block can progress to the point of complete heart block (i.e. where the ventricular rate is slowed). This point would be readily reached if doses of adenosine large enough to cause ischemia detectable with ECG were administered alone without the catecholamine. Since cardiac output is the product of Stroke Volume multiplied by Heart Rate, complete heart block

would greatly increase the risk of cardiovascular collapse. However, not only will the co-administration of synthetic catecholamine reduce the dose of adenosine or adenosinergic agent required to cause detectable myocardial ischemia, and, thus, lessen the chance of complete heart block, the catecholamine will also directly antagonize the heart block producing effect of adenosine.

## VI. POTENTIATING CATECHOLAMINE-INDUCED MYOCARDIAL ISCHEMIA

According to another aspect of the methods for detecting CAD of the present invention, pretreatment with an adenosinergic agent, such as an ATI, is used to potentiate catecholamine-induced myocardial ischemia.

Adenosine transplant inhibitors (ATIs) are reported to increase extracellular levels of adenosine in the myocardium. Therefore, ATIs are capable of producing supply side ischemia. However, as with administration of exogenous adenosine, it is not safe to use large enough doses to induce sufficient myocardial ischemia to be reliably detected by ECG.

It has been reported that administration of large doses of synthetic catecholamines can cause sufficient demand side ischemia to be detected on the ECG (2, 3, 4). However, such large doses of catecholamines can cause dangerous arrhythmias. See, e.g. Goodman and Gilmain's the Pharmacological Basis of Therapeutics, pp. 201 et seq. (8th Ed., 1990)). The risk of serious catecholamine-caused arrhythmia increases with dose. As the dose of catecholamine is increased to induce sufficient myocardial ischemia to be detectable by ECG, catecholamine-caused arrhythmias become an increasingly serious hazard.

The methods of the present invention will reduce the likelihood of such catecholamine-induced arrhythmias by allowing use of smaller doses of catecholamine, while still producing sufficient myocardial ischemia in the presence of CAD to be detectable by ECG. In addition, the side effects (i.e. hypotension, heart block, dizziness, dyspnea, fatigue, and bronchospasm) associated with the use of an adenosinergic agent, such as an ATI, alone will also be substantially reduced, since the dose of adenosinergic agent required for pretreatment of patients before catecholamine administration will be also significantly smaller than the required dose of adenosinergic agent if administered alone.

According to these methods, the intravenous administration of the synthetic catecholamine will be progressive, with stepwise increases. The level of demand ischemia placed on the heart will be assessed by continuous monitoring of the heart rate by ECG. The heart rate at which ischemia occurs will provide prognostic information, i.e. detection of myocardial ischemia which occurred when the heart rate had risen only slightly would indicate more advanced CAD than would detection of myocardial ischemia that occurred near the end of the stepwise catecholamine infusion at a point in the testing protocol when the heart rate had been more substantially elevated.

## VII. PREFERRED ADENOSINERGIC AGENTS

Preferred are adenosinergic agents which have a quick onset and offset of action and consequently have a short duration of action or half-life. Such an activity allows the patient to return to pre-test conditions quickly after the testing protocol is terminated. Also, such an activity profile allows for premature termination of the testing protocol is unacceptable or serious side effects are observed. Thus, preferred are adenosinergic agents having a half life of less than about five minutes. Especially preferred are adenosinergic agents having a half life of less than about one minute.

One preferred class of adenosinergic agents comprise adenosine transport inhibitors (ATI). The ATI's are pharmacological agents which increase local extracellular adenosine levels by preventing transport of adenosine away from that locus.

Another preferred class of adenosinergic agents comprises adenosine kinase inhibitors. Certain adenosine kinase inhibitors are disclosed in the commonly assigned United States Patent Application, Serial No. 647,117, filed January 23, 1991, the disclosure of which is incorporated herein by reference.

## VIII. PREFERRED CATECHOLAMINES

Preferred catecholamines for use according to the methods of the present invention include synthetic catecholamines which elicit specific cardiovascular responses without the adverse effects that would accompany administration of a natural catecholamine. These preferred specific cardiovascular responses similar to those elicited by aerobic exercise and include (a) increased heart rate, (b) increased cardiac output, (c) increased myocardial contractility and (d) increased systolic blood pressure. Thus, preferred synthetic catecholamines include those which elicit increases in heart rate, myocardial contractility, arterial blood pressure, and coronary and skeletal muscle blood flow.

One particularly preferred class of synthetic catecholamines is disclosed in the commonly assigned United States Patent Application, Serial No. 308,683, filed February 9, 1989, the disclosure of which is incorporated herein by reference. These catecholamines include those having the structure depicted in Figure 1, wherein $X_1$ and $X_2$ are independently hydrogen, hydroxy, methoxy or carbamoyl, provides that $X_1$ and $X_2$ are not both bydrogen or carbamoyl; one of $Y_1$ and $Y_2$ is hydrogen and the other is hydrogen or methyl, provides that is $Y_1$ is methyl, then $X_1$ is not carbamoyl; Z is hydrogen or hydroxy and n is 2 or 3, or a pharmaceutically accpetable addition salt thereof.

BIBLIOGRAPHY

1. Beller, G.A., "Pharmacologic stress imaging", JAMA 265: 653-638 (1991).
2. Combs, D.T., Martin, C.M., "Evaluation of isoproterenol as a method of stress testing", Am. Heart J. 87: 711-715 (1974).
3. Wexler, H., Kuaity, J., Simonson, E., "Electrocardiographic effects of isoprenaline in normal subjects and patients with coronary atherosclerosis", Br. Heart J. 33: 759-764 (1971).
4. Mannering, D., Cripps, T., Leech, G., Mehta, N., Valantine, H., Gilmour, S., Bennet, E.D., "The dobutamine stress test as an alternative to exercise testing after acute myocardial infarction", Br. Heart J. 59: 521-526 (1988).
5. Mason, J.R., Palac, R.T., Freeman, M.L., Virupannavar, S., Loeb, H.S., Kaplan, E., Gunnar, R.M., "Thallium scintigraphy during dobutamine infusion: Nonexercise-dependent screening test for coronary disease", Am. Heart J. 107: 481-485 (1984).
6. Beller, G.A.; "Dipyridamole thallium-201 imaging how safe is it?", Circulation 81: 1425-1427 (1990).
7. Pantely, G.A., Bristow, J.D. "Adenosine renewed interest in an old drug", Circulation 82: 1854-1856 (1990).
8. Wilson, R.F., Wyche, K., Christensen, B.V., Zimmer, S., Laxson, D.D; "Effects of adenosine on human coronary arterial circulation", Circulation 82: 1595-1606, (1990).
9. Wackers, F.J., "Adenosine-thallium imaging: Faster and better?", JACC 16: 1384-1386 (1990).
10. Nguyen, T., Heo, J., Ogilby, J.D., Iskandrian, A.S., "Single photon emission computed tomography with thallium-201 during adenosine-induced coronary hyperemia: Correlation with coronary arteriography, exercise thallium imaging and two-dimensional echocardiography", JACC 16: 1375-1383 (1990).

## Claims

1. Use of a synthetic catecholamine in the manufacture of a medicament for use in combination with adenosine or an adenosinergic agent in a method of diagnosing and evaluating coronary artery disease in a mammal, whereby reversible myocardial ischemia is produced when coronary artery disease is present, and evaluating myocardial response.

2. Use of adenosine or an adenosinergic agent in the manufacture of a medicament for use in combination with a synthetic catecholamine in a method of diagnosing and evaluating coronary artery disease in a mammal, whereby reversible myocardial ischemia is produced when coronary artery disease is present, and evaluating myocardial response.

3. Use according to claim 1 or 2 wherein said synthetic catecholamine is a catecholamine which elicits cardiovascular responses similar to those cardiovascular responses elicited by aerobic activity.

4. Use according to claim 3 wherein said catecholamine has the formula:

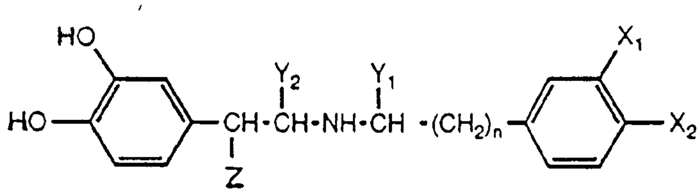

wherein $X_1$ and $X_2$ are independently hydrogen, hydroxy, methoxy or carbamoyl, provided that $X_1$ and $X_2$

are not both hydrogen or carbamoyl; one of $Y_1$ and $Y_2$ is hydrogen and the other is hydrogen or methyl, provided that if $Y_1$ is methyl, then $X_1$ is not carbamoyl; Z is hydrogen or hydroxy and n is 2 or 3, or a pharmaceutically acceptable addition salt thereof.

**5.** Use according to any one of claims 1 to 4 wherein the method of diagnosis comprises administering adenosine.

**6.** Use according to any one of claims 1 to 4 wherein the adenosinergic agent comprises an adenosine transport inhibitor.

**7.** Use according to any one of the preceding claims wherein the method of diagnosing or evaluating coronary artery disease in a patient comprises:

(a) administering for diagnostic purposes a synthetic catecholamine by controlled intravenous infusion to obtain a predetermined elevation in myocardial blood flow requirement;
(b) administrating concurrently a fixed dose of adenosine or an adenosinergic agent having a short half life by intravenous infusion over a selected time interval;
(c) monitoring the patient to detect the presence or absence of myocardial ischemia; and
(d) if myocardial ischemia is not detected in step (c), repeating steps (a) to (c) until either myocardial ischemia is detected or until a preselected maximal myocardial blood flow requirement is reached and wherein with each repeat of step (a) the predetermined elevation in myocardial blood flow amount is increased by a preselected increment.

**8.** Use according to claim 7 wherein electrocardiography is used to monitor the patient to detect the presence of myocardial ischemia.

**9.** Use according to claim 7 wherein elevation of myocardial blood flow requirement is monitored by continuous recording of the patient's heart rate.

**10.** Use according to any one of claims 1 to 4 or 6 wherein the method of diagnosing or evaluating coronary artery disease in a patient comprises:

(a) administering for diagnostic purposes to the patient a preselected dose of an adenosinergic agent;
(b) subsequently administering a synthetic catecholamine by controlled intravenous infusion to obtain a predetermined elevation in myocardial blood flow requirement;
(c) monitoring the patient to detect the presence or absence of myocardial ischemia; and
(d) if myocardial ischemia is not detected in step (c), repeating steps (b) to (c) until either myocardial ischemia is detected or until a preselected maximal myocardial blood flow requirement is reached and wherein with each repeat of step (b), the predetermined elevation in myocardial blood flow requirement is increased by a preselected increment.

**11.** A product containing:

(i) a synthetic catechol amine and
(ii) adenosine or an adenosinergic agent,

as a combined preparation for simultaneous, separate or sequential use in a method of diagnosing and evaluating coronary artery disease in a mammal, whereby reversible myocardial ischemia is produced when coronary artery disease is present and myocardial response is evaluated.

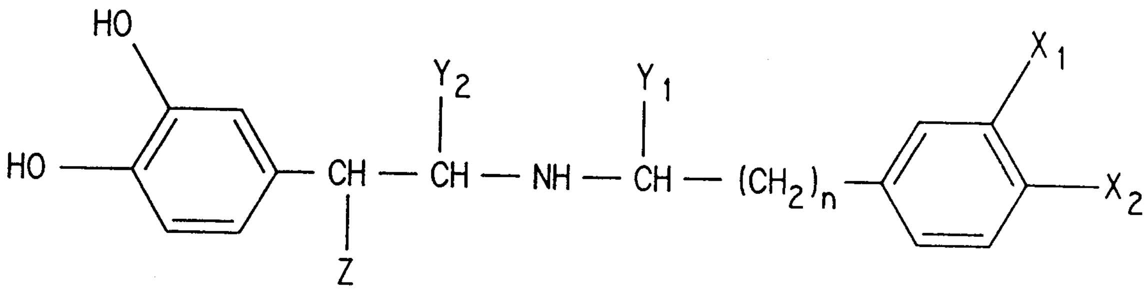

European Patent Office

# EUROPEAN SEARCH REPORT

Application Number
EP 92 31 0384

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y<br><br><br>D | EP-A-0 329 464 (GENSIA PHARMACEUTICALS, INC.)<br>* claims *<br>& US Application 890308683<br>--- | 1-10 | A61K31/70<br>A61K31/505<br>A61K31/165<br>A61K31/135<br>//(A61K31/70, 31:165,31:135)<br>(A61K31/505, 31:165,31:135) |
| Y | EP-A-0 354 638 (MEDCO RESEARCH INC.)<br>* the whole document *<br>--- | 1-10 | |
| Y | CIRCULATION<br>vol. 83, no. 5SUP, May 1991,<br>pages III-2 - III-7<br>A. L'ABBATE 'Pathophysiological basis for noninvasive functional evaluation of coronary stenosis.'<br>* the whole document *<br>--- | 1-10 | |
| X<br><br><br><br><br>Y | STN INTERNATIONAL, KARLSRUHE.<br>FILE BIOSIS. AN=87:129081 BIOSIS. T. TATEDA, 'HEMODYNAMIC EFFECTS OF DOBUTAMINE AND ATP IN ISCHEMIC CANINE HEART'.<br>* abstract *<br>& JPN J ANESTHESIOL 35 (10). 1986. 1505-1512.<br>----- | 11<br><br><br><br><br>1-10 | TECHNICAL FIELDS SEARCHED (Int. Cl.5)<br>A61K |

The present search report has been drawn up for all claims

| Place of search | Date of completion of the search | Examiner |
|---|---|---|
| THE HAGUE | 22 FEBRUARY 1993 | ORVIZ DIAZ P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)